# EUROPÄISCHE PATENTSCHRIFT

(11) **EP 1 592 827 B1**
(45) Veröffentlichungstag und Bekanntmachung des Hinweises auf die Patenterteilung: **16.04.2008**
(21) Anmeldenummer: 04710843.6
(22) Anmeldetag: 13.02.2004
(51) Int. Cl.: D01G 31/00, D01H 13/32

(54) **VORRICHTUNG MIT EINEM MIKROWELLENRESONATOR FÜR EINE ODER AN EINER SPINNEREIVORBEREITUNGSMASCHINE**
DEVICE COMPRISING A MICROWAVE RESONATOR FOR OR ON A SPINNER PREPARATION MACHINE
DISPOSITIF A RESONATEUR MICRO-ONDES POUR OU SUR UNE MACHINE DE PREPARATION A LA FILATURE

(30) Priorität: 13.02.2003 DE 10306209
(43) Veröffentlichungstag der Anmeldung: 09.11.2005
(73) Patentinhaber: Rieter Ingolstadt Spinnereimaschinenbau AG, 85055 Ingolstadt (DE)
(72) Erfinder: GÖHLER, Wolfgang, 85101 Lenting (DE); CHERIF, Chokri, 01309 Dresden (DE)
(74) Vertreter: Schlief, Thomas P.
(86) Internationale Anmeldenummer: PCT/EP2004/001351
(87) Internationale Veröffentlichungsnummer: WO 2004/072337

(56) Entgegenhaltungen:
- EP-A- 1 316 630
- WO-A-00/12974
- US-A- 5 103 180
- US-A1- 2003 150 266
- US-B1- 6 417 676

## Beschreibung

Die Erfindung betrifft eine Vorrichtung mit einem Mikrowellenresonator für eine oder an einer Spinnereivorbereitungsmaschine, die ein Streckwerk zum Verstrecken von strangförmigen Fasermaterial aufweist, insbesondere Karde, Strecke oder Kämmmaschine, wobei der Resonator zum Anschließen an eine Meßeinrichtung zur Messung der Banddichte bzw. der Banddicke und/oder der Feuchtigkeit des kontinuierlich durch den Resonatorraum hindurchgeförderten Fasermaterials ausgebildet ist.

Die Messung von Faserbanddicken ist insbesondere zum Zwecke der Ausregulierung von Ungleichmäßigkeiten von einem oder mehreren einer Spinnereivorbereitungsmaschine vorgelegten Faserbändern unabdingbar. Gleichfalls ist zur Qualitätskontrolle des verstreckten Materials am Maschinenausgang eine derartige Messung wünschenswert. Meßwerte zur Faserbanddichte oder Faserbanddicke (es sind auch die Bezeichnungen Bandquerschnitt oder Bandmasse gebräuchlich; bei Mikrowellenmessungen spricht man meist von Banddichten, d.h. der Bandmasse pro Längeneinheit) werden neben der genannten Qualitätskontrolle auch zum Abstellen der Maschine herangezogen, wenn vorgegebene Masseschwankungsgrenzwerte überschritten werden und somit kein hochwertiges Produkt mehr erhalten wird.

Bisher werden überwiegend mechanisch abtastende Sensoren zur Ermittlung der Banddicke von dem oder den Faserbändern eingesetzt. Auch sind kapazitive Meßorgane bekannt. Eine neue Methode zur Faserbanddichten- bzw. -dickenmessung stellt hingegen die Verwendung von Mikrowellen dar. Hierbei werden von einem Mikrowellengenerator erzeugte Mikrowellen, deren Frequenzen bevorzugt von einem Rechner innerhalb gewisser Grenzen verändert werden, in einen Resonatorraum eines Mikrowellenresonators eingekoppelt, durch welches auch das zu vermessende Fasermaterial kontinuierlich hindurchgeführt wird. Entsprechend der Faserart, der Bandmassen und - geometrie sowie der Bandfeuchtigkeit tritt bei einer charakteristischen Mikrowellenfrequenz ein Resonanzsignal auf, welches nach Auskopplung von einem Rechner zur Ermittlung der Banddichte (Masse pro Längeneinheit) bzw. der Banddicke und/oder der Bandfeuchtigkeit des Fasermaterials auswertbar ist. Eine derartige Methode für andere Anwendungszwecke ist beispielsweise in der EP 0468023 B1 beschrieben, deren Offenbarungsgehalt hiermit explizit eingeschlossen wird. Die Vorteile eines derartigen Meßverfahrens mittels Mikrowellen liegen insbesondere darin, daß hochpräzise, berührungslose Abtastungen des Fasermaterials möglich sind. Mechanische Beeinträchtigungen des Bandes sowie Meßungenauigkeiten aufgrund der Trägheit von mechanischen Meßelementen scheiden aus.

Für die Anordnung eines Mikrowellenresonators ist es naheliegend, diesen dort zu plazieren, wo üblicherweise die mechanisch abtastenden Sensoren angeordnet sind. Am Ausgang beispielsweise eines Streckwerks ist der mechanische Sensor in aller Regel Teil eines Kalanderwalzenpaares, das zum Transport des verstreckten Faserbandes dient. Eine der beiden Kalanderwalzen ist hierbei gegen eine Anpreßkraft auslenkbar ausgebildet, wobei das Maß der Auslenkung ein Maß für die Banddicke des durchlaufenden Faserbandes ist. Da das Kalanderwalzenpaares jedoch insbesondere zum Transport des Faserbandes aus dem Streckwerk dient, kann dieses nicht fortgelassen werden, so daß eine Unterbringung des Mikrowellensensors nicht unproblematisch ist.

WO 00/12974 offenbart eine Vorrichtung mit einem Mikrowellenresonator gemäss dem Oberbegriff des Anspruchs 1.

Es ist Aufgabe der vorliegenden Erfindung, insbesondere bei räumlicher Enge eine einfache Anordnung eines Mikrowellenresonators zu realisieren.

Diese Aufgabe wird bei einer Vorrichtung der eingangs genannten Art dadurch gelöst, daß der Resonator in eine maschinentypische Funktionsgruppe der Spinnereivorbereitungsmaschine integriert ist. Ebenfalls wird die Aufgabe durch eine Spinnereivorbereitungsmaschine mit einer derartigen Vorrichtung gelöst.

Die Vorteile der Erfindung liegen insbesondere darin, daß der Resonator nicht in eventuell vorhandenen Leerräumen untergebracht ist, sondern daß eine an sich bekannte Funktionsgruppe modifiziert wird, um den Resonator darin mechanisch zu integrieren. Der Resonator ist somit Teil der Funktionsgruppe. Hierdurch kann einerseits Platz eingespart werden, andererseits wird eine hochfunktionale Einheit erhalten, die nicht zuletzt auch leicht zugänglich gestaltet werden kann. Da an der bekannten Funktionsgruppe verschiedenste Funktionselemente angeordnet sein können, die gleichfalls beispielsweise zu Wartungs- oder Reinigungszwecken leicht zugänglich sein müssen, ergibt sich dieser Vorteil auch für den Mikrowellenresonator. Der Mikrowellenresonator ist also mit einer Funktionsgruppe der Textilmaschine kombiniert, so daß diese Kombination mehreren Funktionen gleichzeitig dient.

Die Funktionsgruppe mit dem Mikrowellenresonator ist besonders bevorzugt am Streckwerksausgang angeordnet. Bei den herkömmlichen Maschinen ist der Platz zwischen der letzten Streckwerkswalze und dem Kalander- bzw. Abzugsscheibenpaar äußerst begrenzt. Um größere Umkonstruktionen der vorhandenen Maschinen zu vermeiden, wird die Distanz zwischen der genannten Streckwerkswalze und dem Abzugsscheibenpaar bevorzugt unverändert gelassen. Die in diesem Zwischenraum vorhandene Funktionsgruppe umfaßt insbesondere eine Trägerkonstruktion, an der Elemente zur Bandüberwachung, Bandführung und/oder Bandeinfädelung angeordnet sind. Bei der bekannten Strecke RSB-D 35 der Firma Rieter sind an einer solchen Trägerkonstruktion insbesondere eine Vliesführungsdüse angelenkt, die bei Bandstau oberhalb der Trägerkonstruktion von dem gestauten Band verschwenkt wird und damit ein Abstellen der Maschine verursacht. Desweiteren ist eine Halterung in eine zentrale Durchbrechung der Trägerkonstruktion eingesetzt, in den wiederum ein Bandtrichter eingeführt ist. Der Bandtrichter dient zur Verdichtung des von der Vliesführungsdüse zusammengeführten Fasermaterials und zur gezielten Einführung in den Klemmspalt des nachfolgenden Kalanderwalzenpaares. Desweiteren sind bei der bekannten Strecke RSB-D 35 zwei Luftkanäle vorhanden, durch den ein Druckluftstrom geführt wird, um ein in die Vliesführungsdüse eingefädeltes Faserbandende mit Hilfe des Luftstroms durch den Bandtrichter zu den Kalanderwalzen zu transportieren.

Erfindungsgemäß sind nun ein oder mehrere der vorgenannten Elemente auch bei derjenigen Trägerkonstruktion vorhanden, in welcher der Mikrowellenresonator integriert ist. Die genannten Elemente zur Bandüberwachung, Bandführung und/oder Bandeinfädelung können auch eine andere als die vorbeschriebene Ausgestaltung aufweisen.

An der Trägerkonstruktion zwischen dem Streckwerk und der Abzugseinrichtung ist vorzugsweise ebenfalls ein Bandstausensor angeordnet, der insbesondere zur Registrierung eines Bandstaus zwischen einem an der Trägerkonstruktion angeordneten Bandtrichter und der Abzugseinrichtung, d.h. insbesondere einem Kalanderwalzenpaar, dient. Ein solcher Sensor kann auf einem optischen Meßprinzip, auf mechanischer Registrierung oder einem sonstigen Meßprinzip beruhen.

Bevorzugt ist der Resonatorraum zylindrisch und zudem in Faserbandquerrichtung größer auszugestaltet als in Faserbandlaufrichtung. Da - wie vorher erläutert - der Platz zwischen Streckwerk und Abzugseinrichtung bei bekannten Maschinen begrenzt ist, ist eine solche Ausgestaltung vorteilhaft. Insbesondere können Einkoppel- und Auskoppelelemente, die Mikrowellen in den Resonatorraum ein- bzw. auskoppeln bei dieser Ausgestaltung leichter untergebracht und mittels Anschlußelementen angeschlossen werden.

Die Anschlüsse zum Ein- und Auskoppeln der Mikrowellen in den Resonatorraum sind bevorzugt den jeweiligen Bedingungen bzw. Erfordernissen angepaßt angeordnet. So kann es sich anbieten, die Anschlüsse auf der das Fasermaterial zuführenden Seite anzuordnen, da hier einerseits ein leichter Zugang für den Bediener ermöglicht wird und andererseits ein Bandstau an den Kalanderwalzen die Anschlüsse nicht beschädigt. Bei seitlich angeordneten Anschlüssen hingegen läßt sich eine noch kleinere und flachere Baugruppe erhalten.

Der Mikrowellenresonator kann entweder ein selbständiges Teil der Funktionsgruppe sein, oder die Funktionsgruppe und zumindest ein Teil des Resonators sind einstückig ausgebildet. Im letzeren Fall ist es vorteilhaft, wenn der Resonatorraum zumindest teilweise von einer Vertiefung in der Funktionsgruppe gebildet ist. Beispielsweise kann diese von einer gefrästen Ausnehmung gebildet sein. Um den Resonatorraum abzudecken, wird bevorzugt ein abnehmbares Wandelement auf die Vertiefung aufgesetzt und beispielsweise verschraubt. Das Wandelement kann bündig mit der Oberfläche der Funktionsgruppe abschließen oder beispielsweise herausstehen. Ist eine Trägerkonstruktion als Teil der Funktionsgruppe am Streckwerksausgang vorgesehen, dient diese demnach nicht nur beispielsweise zur Aufnahme einer Halterung für einen Bandtrichter und zum Anlenken einer Vliesführungsdüse sowie gegebenenfalls anderer Elemente zur Bandüberwachung, Bandführung und/oder Bandeinfädelung, sondern ist selbst Teil eines Mikrowellensensors.

In einer alternativen Ausgestaltung weist die Funktionsgruppe eine Durchbrechung auf, auf die von der einen und der anderen Seite ein erstes bzw. ein zweites Wandelement aufgesetzt ist. Der Resonatorraum ist hierbei zwischen den beiden Wandelementen gebildet. Diese Ausgestaltung hat unter anderem den Vorteil, daß die beiden Wandelemente leicht abnehmbar ausgestaltet werden können, um beispielsweise Reinigungsarbeiten ausführen zu können. Auch bei Beschädigung oder Abnutzung lassen sich die Wandelemente leicht und kostengünstig auswechseln.

In einer speziellen Ausgestaltung bilden die beiden Wandelemente einerseits die Dekkenwand und andererseits die Bodenwand des Resonatorraums, während dessen umlaufende Seitenwand von der Funktionsgruppe gebildet ist. Alternativ sind auch die Seitenwände des Resonatorraums von einem Abschnitt einer oder beider Wandelemente gebildet.

Zur Hindurchführung des Fasermaterials durch den Resonatorraum weisen die Funktionsgruppe und ein Wandelement bzw. beide Wandelemente miteinander fluchtende Durchgangsöffnungen auf. In diese Durchgangsöffnungen ist besonders bevorzugt ein elektrisch nicht leitendes und an beiden Stirnseiten offenes Rohr eingesetzt, durch das das Fasermaterial hindurchgeführt werden kann. Textilfasern können somit nicht in den übrigen Teil des Resonatorraums eindringen, so daß eine andernfalls von Zeit zu Zeit notwendige Reinigung hinfällig werden kann.
Das Führungsrohr ist bevorzugt aus einem im wesentlichen temperaturstabilen Material, dessen Dielektrizitätskonstante zudem im wesentlichen temperaturunabhängig ist gefertigt. Es bietet sich an, hierzu Keramik, einen Verbundwerkstoff oder Kunststoff zu verwenden, im letzteren Fall insbesondere Polykarbonat. Zudem so*llen die Mikrowellenmessungen möglichst nicht beeinflußt werden. Weiterhin ist es von Vorteil, wenn das verwendete Material möglichst wenig Feuchtigkeit aufnimmt. Als Verbundwerkstoff hat sich insbesondere TMM^{®} der amerikanischen Firma Rogers als geeignet erwiesen, das ein Hydrokarbon-Keramik-Verbundwerkstoff mit einer sehr guten Temperaturstabilität und insbesondere einer gegenüber Temperaturschwankungen sehr stabilen Dielektrizitätskonstanten ist.

Das Führungsrohr ist in einer bevorzugten Variante zumindest abschnittsweise in Faserbandlaufrichtung konisch bzw. trichterförmig ausgebildet, um das Fasermaterial für ein nachfolgendes Kalanderwalzenpaar schon zu einem bestimmten Grad zu verdichten.

Zum Zweck einer einfacheren Einfädelung des Fasermaterials in den Resonator kann die Eintrittsöffnung des Führungsrohres erweitert und insbesondere konisch ausgebildet sein.

Da das Führungsrohr bevorzugt auswechselbar ausgestaltet ist, kann beispielsweise je nach Bandfeinheit ein passendes Führungsrohr mit modifiziertem Innendurchmesser gewählt werden. Zweckmäßigerweise ist die Auswertung der ausgekoppelten Mikrowellensignale auf das jeweils verwendete Führungsrohr abzustimmen. Ein derartiger Neuabgleich der Auswertungssoftware kann sich erübrigen, wenn die Massen der verschiedenen Führungsrohre im Bereich der Mikrowellenausbreitung im wesentlichen gleich groß gewählt werden. Dies bedeutet eine entsprechend geeignete Geometriewahl der Rohre.

Die vorstehende Ausführungsform betrifft den Fall, daß lediglich jeweils ein Rohr den Resonatorraum durchsetzt, d.h. der Außendurchmesser der jeweiligen Rohre ist bei unterschiedlichem Innendurchmesser gleich. Bei alternativen Ausführungsformen sind mindestens zwei Führungsrrohre vorgesehen, wobei ein inneres in einem äußeren angeordnet, beispielsweise eingeschoben, ist. Das Fasermaterial wird hierbei im inneren Rohr geführt, während das äußere Führungsrohr nicht unbedingt mit dem Fasermaterial in Berührung kommt. In diesem Fall können ebenfalls verschiedene innere Führungsrohre mit verschiedenen Innendurchmessern wechselweise in das vorzugsweise selbe äußere Rohr eingesetzt werden. Das äußere Rohr dient vornehmlich zur Halterung an der Trägerkonstruktion und zur Aufnahme des inneren Rohres. Die Genauigkeit der Resonanzsignalauswertung für die verschiedenen inneren Rohre kann bei dieser Ausführungsform ebenfalls durch Neuabgleich der Auswertungssoftware und/oder durch gleiche Masse der inneren Rohre im Mikrowellenausbreitungsbereich gewährleistet werden.

In einer Weiterführung des Gedankens mindestens zweier ineinander gesteckter Rohre kann das äußere Rohr mit durchgehender Rohrwandung ausgebildet sein, während das innere Rohr Durchbrechungen aufweist, beispielsweise ein oder mehrere Löcher. Auf diese Weise kann z.B. Druckluft in den Zwischenraum zwischen den beiden Rohrwandungen eingeführt werden, welches durch das bzw. die Löcher eintritt und als Einfäde-Ihilfe und/oder Förderhilfe für das durch den Resonatorraum zu führende Faserband dient. Auch kann auf diese Weise bei nicht vorhandenem Faserband das innere Rohr gesäubert werden.

Prinzipiell ist es von Vorteil, eine Blas- oder Saugeinrichtung an dem Resonator vorzusehen, um das oder die Faserbänder durch den Resonatorraum zu fördern und/oder diesen sauber zu halten.

Vorteilhafterweise ist das Führungsrohr an seiner stromabwärtigen Seite mit einem Bandtrichter gekoppelt bzw. koppelbar, so daß diese Einheit eine Doppelfunktion bei minimalem Platzaufwand einnehmen kann. Bei einer direkten Verbindung miteinander kann der Bandtrichter an das Rohrende beispielsweise aufgesteckt, mit diesem verschraubt oder anderweitig verbunden sein. Alternativ ist auch eine einstückige Ausbildung von Führungsrohr und Bandtrichter möglich. Der Bandtrichter legt das verstreckte Faserband bevorzugt einem Klemmspalt eines nachfolgenden Kalanderwalzenpaares vor, so daß die freie vom Faserband zurückgelegte Wegstrecke zwischen Bandtrichter und Kalanderwalzenpaar möglichst kurz ist.

Bei einer Alternative ist der Bandtrichter an der Trägerkonstruktion oder einem abnehmbaren Wandelement anordenbar, ohne daß eine Verbindung zu dem Führungsrohr bestünde.

Da sich herausgestellt hat, daß die Wärmeausdehnung der Resonatorwände zu Meßverfälschungen führen kann, ist bevorzugt Sorge zu tragen, daß im wesentlichen konstante Temperaturverhältnisse gegeben sind. Eine Möglichkeit besteht darin, die Funktionsgruppe im Bereich des Resonators beheizbar auszubilden, wozu beispielsweise eine mit elektrischer Spannung beaufschlagbare Heizfolie auf die Funktionsgruppe aufgebracht werden kann, um eine gewünschte, konstante Temperatur schnell zu erreichen. Eine solche Folie kann zweckmäßigerweise an der Unter- und/oder Oberseite des Resonatorraums plaziert werden. Bei der oben beschriebenen Ausgestaltung des Resonatorraums unter Verwendung eines oder mehrerer Wandelemente können auch diese beheizt werden. Die Regelung des Temperaturniveaus kann beispielsweise von der Mikrowellenelektronik, einer zentralen Maschinensteuerung oder einer eigens hierfür zuständigen Regelung vorgenommen werden. Beim Erreichen der gewünschten Temperatur bzw. kurz vorher wird der Stromkreis geöffnet, während beim Unterschreiten einer vorgegebenen Temperaturgrenze der Stromkreis wieder geschlossen wird.

Statt einer Erwärmung können Elemente der Vorrichtung auch auf eine konstante Temperatur gekühlt werden, beispielsweise mittels Peltier-Elementen.

Alternativ oder zusätzlich werden Materialien mit geringer Wärmeausdehnung für die Funktionsgruppe bzw. zumindest für den Resonator verwendet. Eine vorteilhafte Ausgestaltung ist hierbei die Verwendung von einem Stahl mit vorzugsweise hohem Nickel-Anteil, insbesondere Ni 36-Stahl. Ein derartiger Stahl ist beispielsweise Invar®-Stahl, der einen sehr geringen Wärmeausdehnungskoeffizienten besitzt.

Alternativ oder zusätzlich werden thermisch isolierende Materialien an der Funktionsgruppe angeordnet, um ein konstantes Temperaturniveau zu erhalten. In einer speziellen Ausgestaltung wird thermisch isolierendes Material in die Funktionsgruppe integriert. Ist beispielsweise der Resonator als separates Teil der Funktionsgruppe ausgebildet und in eine entsprechende Durchbrechung der Funktionsguppe bzw. einer Trägerkonstruktion eingesetzt, kann an den Kontaktstellen ein thermisch isolierendes Material angeordnet sein, um den Wärmeübergang von dem benachbarten Abschnitt der Funktionsgruppe, die üblicherweise aus Stahl gefertigt ist, zum Resonator zu minimieren.

Alternativ oder zusätzlich kann der Resonator mitsamt der Funktionsgruppe in einem thermisch isolierenden Gehäuse untergebracht sein. Durchgänge-für das Fasermaterial sind selbstverständlich vorzusehen.

Bei der Verwendung von Materialien geringer thermischer Ausdehnung, wie beispielsweise einem Stahl mit vorzugsweise hohem Nickel-Anteil, kann die Ausbreitungsfähigkeit des Mikrowellenfeldes im Resonator leiden. Dementsprechend besteht eine bevorzugte Ausführungsform der Erfindung darin, daß der Resonatorraum innenwandig mit einer leitfähigen Beschichtung versehen ist, die beispielsweise von einer sauerstoffarmen Kupfer-Beschichtung gebildet ist.

Um eine Korrosion der inneren Oberflächen des Resonatorraums zu verhindern, kann es vorteilhaft sein, eine korrosionsbeständige Beschichtung aufzubringen, die beispielsweise aus Gold oder Silber besteht. Dies ist insbesondere dann zweckmäßig, wenn die zuvor genannte leitfähige Beschichtung aus beispielsweise Kupfer verwendet wird. In diesem Fall wird die Korrosion des Kupfers durch das Aufbringen einer Gold-oder Silber-Beschichtung verhindert.

Alternativ oder zusätzlich ist der Resonatorraum gasdicht mit einem vor Korrosion schützenden Gas gefüllt, welches dazu insbesondere keinen Sauerstoff enthalten darf. Es bietet sich hierbei insbesondere die Verwendung von Edelgas an.

Bestandteil der Erfindung ist neben der beschriebenen Vorrichtung auch eine Spinnereivorbereitungsmaschine, insbesondere Karde, Strecke oder Kämmmaschine, welche die erfindungsgemäße Vorrichtung aufweist. Gleichfalls ist Bestandteil der Erfindung das vorbeschriebene Führungsrohr zum austauschbaren Einsetzen in einen Mikrowellenresonator der zuvor beschriebenen Vorrichtung.

Vorteilhafte Weiterbildungen der Erfindung sind durch die Merkmale der Unteransprüche gekennzeichnet.

Im folgenden wird die Erfindung anhand der Figuren näher erläutert. Es zeigen:
- Figur 1: eine schematische Seitenansicht einer Strecke gemäß dem Stand der Technik;
- Figur 1a, 1b: eine Funktionsgruppe zwischen Streckwerksausgang und Kalanderwalzen in Vergrößerung (in Seitenansicht und in perspektivischer Ansicht);
- Figur 2: eine erfindungsgemäße Funktionsgruppe mit einem Mikrowellenresonator in geschnittener Vorderansicht (Schnitt entlang II-II in Figur 4);
- Figur 3: die Funktionsgruppe gemäß der Figur 2 in geschnittener Seitenansicht (Schnitt entlang I-I in Figur 4);
- Figur 4: eine Aufsicht auf die Funktionsgruppe gemäß der Figuren 2 und 3 (in kleinerem Maßstab);
- Figur 5: eine geschnittene Seitenansicht einer Funktionsgruppe gemäß einer zweiten Ausführungsform;
- Figur 6: eine geschnittene Seitenansicht einer Funktionsgruppe gemäß einer dritten Ausführungsform;
- Figur 7: eine geschnittene Seitenansicht einer Funktionsgruppe gemäß einer vierten Ausführungsform;
- Figur 8: eine geschnittene Seitenansicht einer Funktionsgruppe gemäß einer fünften Ausführungsform mit zwei Heizfolien;Figur 9 eine Detailansicht aus der Figur 8;
- Figur 10: eine geschnittene Detailansicht einer speziellen Ausführungsform zweier ineineinander geschobener, als Dielektrika ausgebildeter Rohre, und
- Figur 11: eine geschnittene Detailansicht einer weiteren Ausführungsform zweier ineineinander geschobener Rohre.

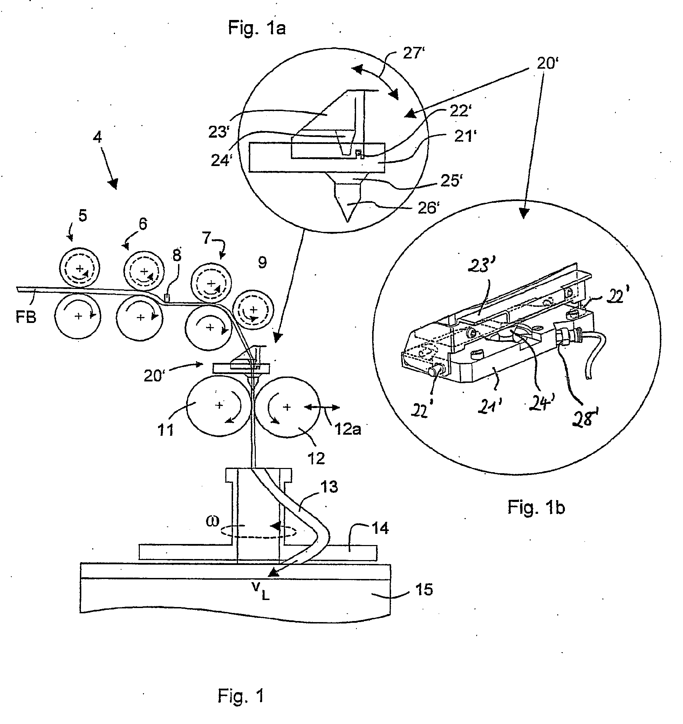

In der Figur 1 sind wesentliche Elemente einer bekannten Strecke dargestellt. Das Herzstück einer solchen Strecke ist das Streckwerk 4, welches in der dargestellten Ausführungsform ein Eingangswalzenpaar 5, ein Mittelwalzenpaar 6 und ein Ausgangs- bzw. Lieferwalzenpaar 7 aufweist, die sich mit in dieser Reihenfolge jeweils gesteigerter Umfangsgeschwindigkeit drehen. Ein oder mehrere Faserbänder FB werden zwischen den jeweiligen Walzen der Walzenpaare 5, 6, 7 geklemmt und entsprechend dem Verhältnis der Umfangsgeschwindigkeiten der Walzenpaare verzogen. Im Hauptverzugsfeld, das vom mittleren Walzenpaar 6 und dem Lieferwalzenpaar 7 gebildet ist, ist zusätzlich ein Druckstab 8 zur Umlenkung des Faserbandes FB angeordnet. Das oder die verzogenen Faserbänder FB werden mit Hilfe einer Umlenkoberwalze 9 in mehreren Bandführungselementen (siehe Figuren 1a, 1b) zusammengefaßt und über ein Kalanderwalzenpaar 11, 12 in einen geschwungenen Bandkanal 13 eingeführt. Der Bandkanal 13 ist in einem sich mit der Winkelgeschwindigkeit ω drehenden Drehteller 14 angeordnet, wodurch das Faserband FB mit einer Geschwindigkeit v_{L} in einer rotierenden oder changierenden Kanne 15 abgelegt wird. Die Kalanderwalze 12 bei dieser bekannten Strecke ist ortsbeweglich und kraftbeaufschlagt ausgebildet (siehe Doppelpfeil 12a), wobei die Größe der Auslenkung ein Maß für den Bandquerschnitt des zwischen den Kalanderwalzen 11, 12 durchlaufenden Faserbandes FB ist. Die Signale bezüglich der Auslenkung werden an eine nicht dargestellte Auswerteeinheit weitergegeben, die zur Berechnung der Faserbandqualität dient. Bei Über- oder Unterschreiten von Grenzwerten des Bandquerschnitts kann die Maschine automatisch abgestellt werden.

In den Figuren 1a und 1b sind die Bandführungselemente der bekannten Funktionsgruppe 20' zwischen dem Streckwerk 4 und den Kalanderwalzen 11, 12 vergrößert dargestellt. Die schematisch gezeigte Ausführungsform gemäß der Figur 1a entspricht derjenigen der bekannten Strecke RSB D30 der Firma Rieter. Zentrales Element ist hierbei eine plattenförmig ausgebildete Trägerkonstruktion 21'. Die Trägerkonstruktion 21' weist seitliche Lagerbolzen 22' zur Anlenkung einer verschwenkbaren Vliesführungsdüse 23' (auch Vliestrichter genannt), so daß diese oberhalb der Trägerkonstruktion 21' im Falle eines Bandstaus an der Düse 23' nach vorne verschwenkt werden kann (siehe Doppelpfeil 27'). Die Vliesführungsdüse 23' formt das aus dem Streckwerk 4 kommende Vlies bzw. Faserband FB zu einem festeren Band. In die Vliesführungsdüse 23' ist ein Vliesdüseneinsatz 24' eingesetzt, durch welchen das Faserband FB hindurchgeführt wird. In Bandtransportrichtung schließt sich eine in der Trägerkonstruktion gehaltene Halterung 25' an, in die ein Bandtrichter 26' mit einem stromabwärtigen, schnabelförmigen Endabschnitt eingesetzt ist.

Der Figur 1b ist desweiteren zu entnehmen, daß ein Druckluftanschluß 28' an der Trägerkonstruktion 21' vorgesehen ist, um ein Faserbandende leichter in die Vliesführungsdüse 23', den Vliesdüseneinsatz 24' und den Bandtrichter 26' einfädeln zu können.

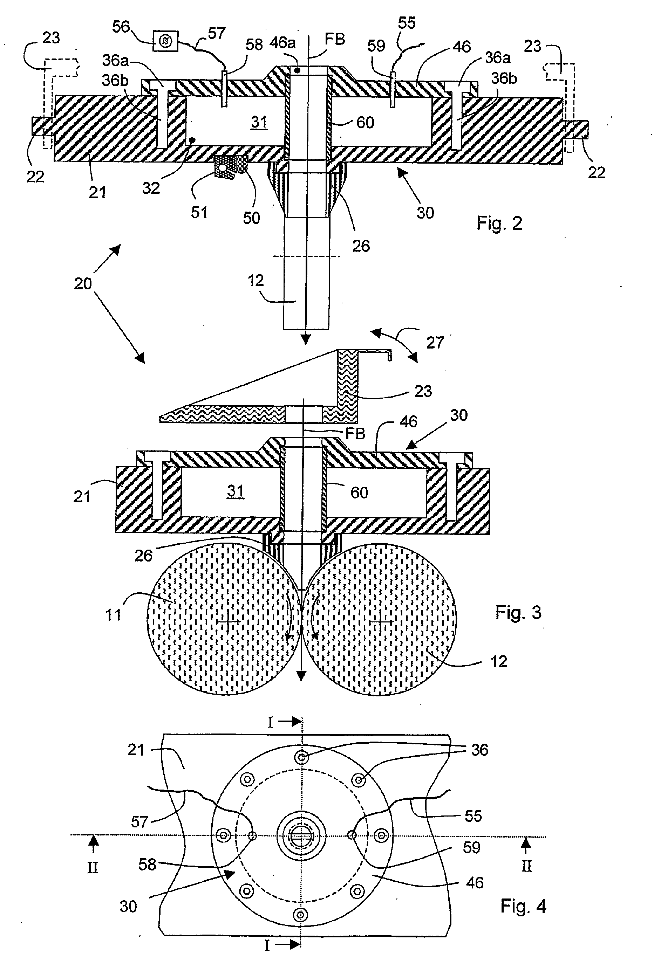

In den Figuren 2 und 3 ist ein erstes Ausführungsbeispiel der Erfindung von vorne und von der Seite jeweils im zentralen Schnitt dargestellt. Eine Funktionsgruppe 20 umfaßt eine plattenförmige Trägerkonstruktion 21, an der zu zwei gegenüberliegenden Seiten - wie auch im Stand der Technik - Anlagebolzen 22 zum Aufsetzen einer gemäß dem Doppelpfeil 27 verschwenkbaren Vliesführungsdüse 23 angeordnet sind (s. Figur 3, in den Figuren 2 und 4 nicht dargestellt). Die Trägerkonstruktion 21 weist eine zentrale Vertiefung 32 auf, die in der dargestellten Ausführungsform (siehe auch Figur 4) zylinderförmig ausgebildet ist. An der Unterseite der Trägerkonstruktion 21 ist ein optischer Sensor 50 in einer Ausnehmung geringer Tiefe angeordnet, wobei die Sensoroberfläche mittels einer Druckluft abgebenden Reinigungsvorrichtung 51 von Faserflug freigehalten wird.

Desweiteren ist im Bereich einer Durchgangsöffnung an der Unterseite der Trägerkonstruktion 21 ein im wesentlichen die Gestalt eines Hohlschnabels aufweisender Bandtrichter 26 angeordnet, der beispielsweise mittels einer nicht näher dargestellten Schraubverbindung an der Trägerkonstruktion 21 lösbar befestigt ist. Andere Befestigungsmöglichkeiten des Bandtrichters 26 sind ebenfalls möglich; so kann beispielsweise auch ein zusätzliche Halterung zwischen Bandtrichter 26 und Trägerkonstruktion 21 angeordnet sein.

Auf die Vertiefung 32 ist ein Wandelement 46 aufgesetzt, das in der dargestellten Ausführungsform als flache Zylinderscheibe ausgebildet ist und randseitig Schraubenaufnahmen 36a aufweist, die mit entsprechenden Sackbohrungen 36b in der Trägerkonstruktion 21 fluchten. Wie in der im gegenüber den Figuren 2 und 3 in kleinerem Maßstab wiedergegebenen Figur 4 dargestellt, können in diese Bohrungen 36a, 36b, welche jeweils Innengewinde aufweisen, Sechskantschrauben 36 eingeschraubt werden, um das Wandelement 46 mit der Trägerkonstruktion 21 zu verschrauben (die Schrauben sind in den Figuren 2 und 3 nicht dargestellt). In einer nicht dargestellten Alternative kann das Wandelement 46 in einer Ausnehmung in der Trägerkonstruktion 21 planparallel mit der Oberseite der Trägerkonstruktion 21 eingepaßt und verschraubt sein.

Das auf die Vertiefung 32 aufgesetzte Wandelement 46 läßt einen Resonatorraum 31 eines Mikrowellenresonators 30 entstehen, in den Mikrowellen mit Hilfe eines Einkoppelelements 58 eingekoppelt und mit Hilfe eines Auskoppelelements 59 ausgekoppelt werden können. Beide Koppelelemente 58, 59 ragen durch entsprechende Bohrungen in dem Wandelement 46 von außen in den Resonatorraum 31. Das Einkoppelelement 58 ist über ein Kabel 57 an einen schematisch angedeuteten Mikrowellengenerator 56 angeschlossen, dessen Frequenz mit Hilfe einer nicht dargestellten Steuereinheit (vorzugsweise ein Mikroprozessor) variiert werden kann. Das Auskoppelelement 59 ist seinerseits über ein Kabel 55 mit einer nicht dargestellten Auswerteeinheit verbunden. Das Auskoppelelement 59 empfängt die im Resonator sich ausbildenden Mikrowellensignale und leitet sie an die Auswerteeinheit weiter, so daß diese zu aufeinander folgenden Zeitpunkten die jeweilige Resonanzfrequenz und die dazugehörige Signalbreite ermittelt werden kann. Aus diesen Informationen kann dann die Banddicke bzw. Bandmasse (pro Längeneinheit) des jeweils gerade den Resonatorraum durchlaufenden Fasermaterials ermittelt werden.

In die Vertiefung 32 eingesetzt ist ein hohlzylinderförmiges Führungsrohr 60 aus dielektrischem Material, das an seinen beiden Stirnseiten in entsprechenden stufenförmigen Aufnahmen in der Trägerkonstruktion 21 bzw. dem Wandelement 46 lagert. Die Durchgangsöffnung im Führungsrohr 60 fluchtet mit zentralen Durchgangsöffnungen im Wandelement 46, in der Trägerkonstruktion 21 und im Bandtrichter 26. Das Faserband FB (nur schematisch als Pfeil dargestellt) kann somit linear durch die Funktionsgruppe 20 direkt in den Klemmspalt zwischen den beiden Kalanderwalzen 11, 12 geführt werden (siehe insbesondere Figur 3).

Das Führungsrohr 60 ist in einfacher Weise auszuwechseln, indem das Wandelement 46 losgeschraubt und abgenommen wird. Je nach Materialart und Verstreckungsbedingungen können verschiedene Einsätze 60 verwendet werden.

Zwischen der Vliesführungsdüse 23 und dem Wandelement 46 kann ein weiteres, hier nicht dargestelltes Bandführungselement angeordnet sein, ähnlich dem Vliesführungsdüseneinatz 24 gemäß dem Stand der Technik der Figur 1.

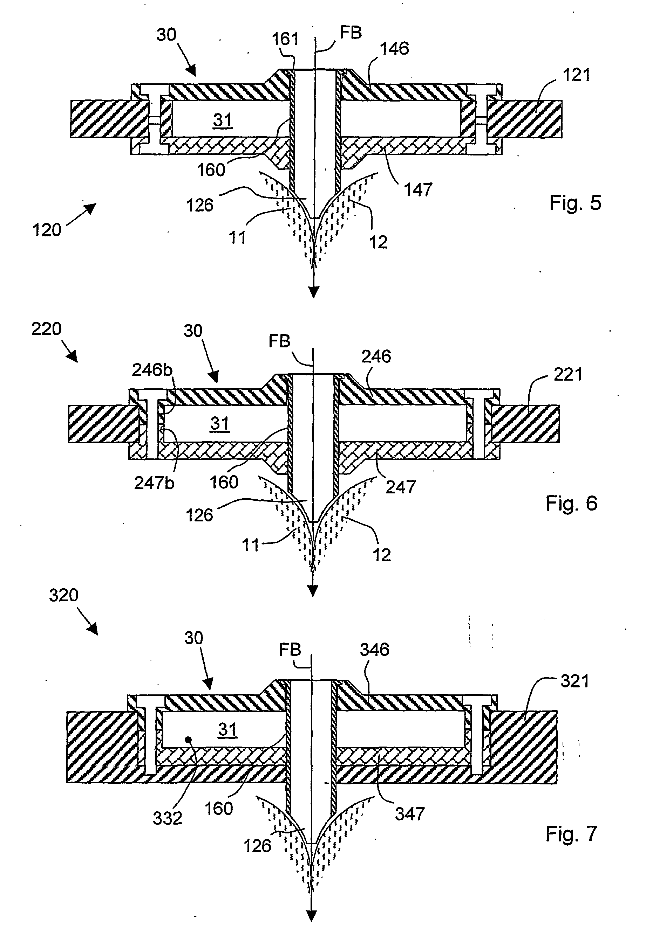

In den Figuren 5 bis 8 sind weitere Ausführungsformen der Erfindung in geschnittener Seitenansicht wiedergegeben. Die Kalanderwalzen 11, 12 sind hier jeweils nur angedeutet.

Bei der Ausführungsform einer Funktionsgruppe 120 gemäß der Figur 5 bedecken den Resonatorraum 31 von der einen Seite ein Wandelement 146 und von der anderen Seite ein Wandelement 147. Die Wandelemente 146, 147 bilden hierbei die Decke bzw. den Boden des Resonatorraums 31, während die umlaufende Seitenwand von der Trägerkonstruktion 121 gebildet ist. Die Wandelemente 146, 147 sind mit der Trägerkonstruktion 121 mittels Schrauben (es sind wiederum nur die entsprechenden, jeweils Innengewinde aufweisenden Bohrungen dargestellt) verbunden. Statt zweier sich jeweils gegenüberliegender Schrauben ist es selbstverständlich in einer nicht dargestellten Variante auch möglich, Schrauben lediglich von einer Seite her einzuführen, die in korrespondierende Sackbohrungen im anderen Wandelement enden.

Die Trägerkonstruktion 121 kann durchgehend ausgebildet sein und - in Aufsicht betrachtet - den Resonator 30 allseitig umgeben (analog der in Figur 4 gezeigten Ausführung). In einer nicht dargestellten Variante ist die Trägerkonstruktion 121 hingegen zweiteilig ausgebildet, wobei - wiederum in Aufsicht gesehen - die beiden Teile durch die Wandelemente 146, 147 miteinander verbunden sind. Die Wandelemente 146, 147 bilden hierbei eine Art Brücke, in welcher der Resonator 30 untergebracht ist.

Gemäß der Ausführungsform einer Funktionsgruppe 220 der Figur 6 bilden zwei Wandelemente 246, 247 allein, d.h. ohne Beteiligung von Innenflächen der Trägerkonstruktion 221, den Resonatorraum 31. Hierzu weisen die beiden Wandelemente 246, 247 zueinander gerichtete Umfangswulste 246b, 247b auf, in denen miteinander fluchtende Bohrungen zur Einführung von Schrauben (nicht dargestellt) vorgesehen sind. Der Resonator 30 kann - wie zuvor für die Ausführungsform gemäß der Figur 5 beschrieben - in einer einteiligen Trägerkonstruktion 221 eingebettet sein, oder die Wandelemente 246, 247 verbinden zwei oder mehrere Teile einer geteilten Trägerkonstruktion 221.

Die in der Figur 7 dargestellte, vierte Ausführungsform einer Funktionsgruppe 320 zeichnet sich dadurch aus, daß ein Wandelement 347 in eine Vertiefung 332 in der Trägerkonstruktion 321 eingesetzt ist und auf dieses Wandelement 347 ein weiteres Wandelement 346 aufgesetzt und mittels Schrauben (nicht dargestellt) verschraubt ist. Auch bei dieser Ausführungsform ist der Resonatorraum 31 lediglich von den beiden Wandelementen 346, 347 umschlossen. Die Trägerkonstruktion 321 kann - ebenso wie im Falle der Figur 221 - aus einem geeigneten Metall bzw. einer Metalllegierung oder aus einem Kunststoff bestehen. Im letzteren Fall kann der Kunststoff thermisch isolierend wirken.

Die in den Figuren 5 bis 7 dargestellten Ausführungsformen zeigen ein Führungsrohr 160, an welchem einstückig ein Bandtrichter 126 mit einem stromabwärtigen, schnabelförmig ausgebildeten Endabschnitt angeformt ist, der das Faserband FB zwischen die - nur im Ausschnitt angedeuteten - Kalanderwalzen 11, 12 legt. Das Führungsrohr 160 weist an seinem einen den Kalanderwalzen 11, 12 abgewandten Ende eine Umfangswulst 161 auf, die bei eingesetztem Führungsrohr 160 an einem entsprechend gestuften Rand der Durchgangsöffnung des oberen Wandelements 146, 246 bzw. 346 zur Anlage kommt. Auf diese Weise ist einerseits eine sichere Lagerung des Führungsrohrs 160 im Resonator 30 sichergestellt, andererseits kann das Führungsrohr 160 schnell und problemlos gewechselt werden.

In nicht weiter dargestellten Ausführungsformen können die Trägerkonstruktion 21, 121, 221, 321 derart ausgebildet sein, daß die Wandelemente 146, 147, 246, 247, 346 bündig mit der Trägerkonstruktion 21, 121, 221, 321 abschließen.

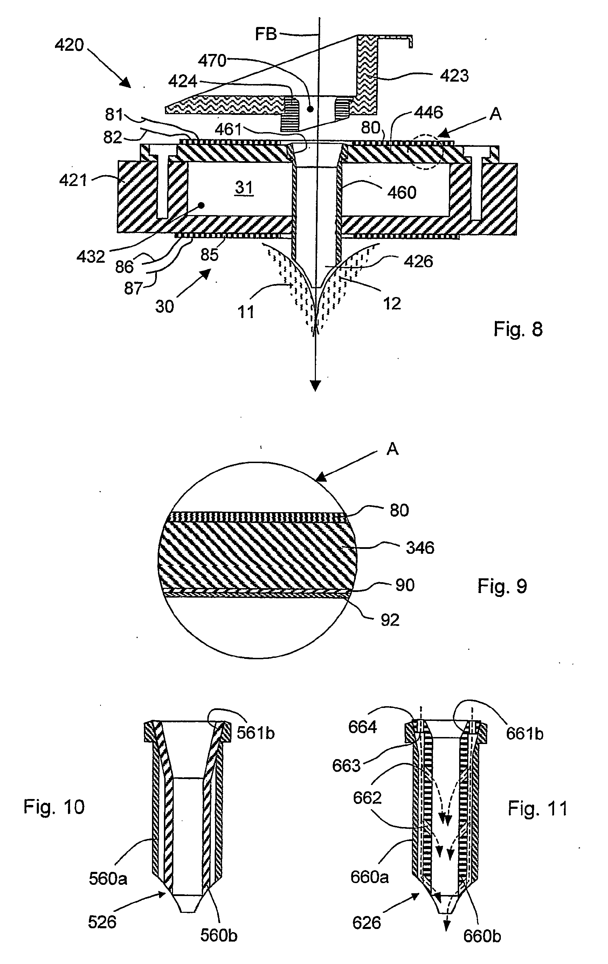

In der Figur 8 ist eine Ausführungsform einer Funktionsgruppe 420 dargestellt, die derjenigen der Figur 2 bis 4 ähnelt (s. insbes. Figur 3). Wiederum bedeckt ein abnehmbares Wandelement 446 eine Vertiefung 432 und ist mit nicht näher dargestellten Schrauben mit einer Trägerkonstruktion 421 verschraubbar. Auf der dem Resonator 30 abgewandten Seite des Wandelements 446 ist eine erste, elektrische Heizfolie 80 angebracht, während auf der gegenüberliegenden Seite der Trägerkonstruktion 421 außenseitig eine zweite Heizfolie 85 angeordnet ist. Beide Heizfolien 80, 85 sind über Anschlußdrähte 81, 82 bzw. 86; 87 mit einer nicht dargestellten Heizquelle verbunden. Die Heizleistung wird vorteilhafterweise geregelt, beispielsweise auf 35° C. Hierfür sind zweckmäßigerweise ein oder mehrere nicht dargestellte Temperaturmeßeinrichtungen vorgesehen, die beispielsweise in einer bzw. mehreren, nahe an den Resonatorraum 31 heranreichenden, seitlichen Bohrungen in der Trägerkonstruktion 421 angeordnet sein können. Eine thermische Hüllisolation, welche beispielsweise die gesamte Trägerkonstruktion - mit entsprechenden Öffnungen für das Fasermaterial - umgibt, kann zur Verhinderung des Einflusses von Temperaturschwankungen in der Umgebung sowie zur Verhinderung eines Heizleistungsverlustes gleichfalls vorgesehen sein.

Weitere zusätzliche oder alternative Maßnahmen zur Temperaturstabilisierung können darin bestehen, daß die den Resonatorraum 31 umgebenden Elemente bei allen dargestellten Ausführungsformen aus einem Material mit geringer Wärmeausdehnung gefertigt sind, beispielsweise aus Invar®-Stahl. Zusätzlich können auch andere Elemente der gesamten Funktionsgruppe aus einem solchen Material gefertigt sein.
Entsprechend der mit "A" bezeichneten Ausschnittsvergrößerung der Figur 9, weist die Innenwandung des Resonators 30 bei der Ausführungsform gemäß der Figur 8 eine leitfähige Beschichtung 90 aus beispielsweise sauerstoffarmem Kupfer auf, da der Invar®-Stahl des Wandelements 446 und der Trägerkonstruktion 421 eine nur relativ geringe elektrische Leitfähigkeit besitzt. Mikrowellenresonanzen mit genügender Signalstärke könnten sich ohne eine solche leitfähige Beschichtung ggf. nicht ausbilden. Um eine Korrosion der Beschichtung 90 zu verhindern, ist auf dieser zusätzlich eine korrosionsbeständige Beschichtung 92 aus beispielsweise Gold oder Silber aufgebracht. Alternativ kann eine Keramik oder ein Verbundstoff mit eingebetteter Keramik als Beschichtung oder Abdeckung eingesetzt werden.

In den Resonatorraum 31 der Ausführungsform gemäß der Figur 8 ist ein Führungsrohr 460 eingesetzt, welches im Bereich des Wandelements 446 eine konische Erweiterung 461 aufweist. Die gegenüberliegende Stirnseite ist als Bandtrichter 426 ausgebildet (vgl. Figuren 5 bis 7), der das Faserband FB möglichst nah in den Klemmspalt zwischen den Kalanderwalzen 11,12 vorlegt.

Oberhalb des Wandelements 446 ist eine Vliesführungsdüse 423 angeordnet (vgl. Figur 3), die eine Bohrung 470 aufweist, in die ein Vliesdüseneinsatz 424 eingesetzt und mittels einem nicht dargestellten Zentrierstift gehalten ist. Auf der dem Führungsrohr 460 abgewandten Seite ist der Vliesdüseneinsatz 24 umfangseitig gerundet ausgebildet, um ein schonendes Einführen des Faserbandes FB in das Führungsrohr 460 zu gewährleisten.

In den Figuren 10 und 11 ist jeweils ein inneres Führungsrohr 560b bzw. 660b (mit jeweilig konischen Erweiterungen 561 b bzw. 661 b) in ein äußeres Rohr 560a bzw. 660a eingeschoben, wobei die jeweilige Kombination beispielsweise in einen Resonator 30 gemäß der Figur 8 einsetzbar ist. An den äußeren Führungsrohren 560a bzw. 660a ist unterseitig jeweils ein Bandtrichter 526 bzw. 626 angeordnet, der jedoch auch an den inneren Führungsrohren 560b bzw. 660b vorgesehen sein könnte. Diese beiden Ausführungsformen haben den Vorteil, daß das äußere Rohr 560a bzw. 660a im Resonator eingesetzt verbleiben kann, während das innere Rohr 560b bzw. 660b gegen ein solches mit kleinerem oder größerem Innendurchmesser ersetzt werden kann. Auf diese Weise kann den jeweiligen Bandeigenschaften (z.B. schmalerer oder breiterer Banddurchmesser; Textilmaterialart, o.ä.) Rechnung getragen werden. Vorteilhafterweise sind hierbei die Massen der verschiedenen inneren Führungsrohre 560b bzw. 660b im Bereich der Mikrowellenausbreitung im wesentlichen gleich groß, so daß kein Neuabgleich des Resonators 30 bei Auswechseln des inneren Rohres 560b bzw. 660b durchgeführt werden muß.

Bei der Ausführungsform gemäß der Figur 11 weist das äußere Rohr 660a (wie das Rohr 560a gemäß der Figur 10) eine durchgehende Rohrwandung auf, während das innere Rohr 660b Durchbrechungen 662 in Form mehrerer über den Umfang verteilter und in Längsrichtung beabstandeter Löcher aufweist. Auch an der oberseitigen Wulst 664, die von oben gesehen kreisförmig ausgebildet ist, sind Durchbrechungen 663 vorgesehen, durch welche Blas- bzw. Druckluft aus einer nicht dargestellten Druckluftquelle in den Raum zwischen der Innenwandung des äußeren Rohres 660a und der Außenwandung des inneren Rohres 660b einleitbar ist. Die Druckluft gelangt dann über die Durchbrechungen 662 in das Innere des Rohres 660b (s. gestrichelte Pfeile) und kann dort zur Einfädelung und/oder Hindurchförderung des Faserbandes durch den Resonator dienen. Alternativ oder zusätzlich kann die Druckluft zum Säubern des genannten Zwischenraumes zwischen den beiden Rohren sowie des Innenraums des inneren Rohres 660b eingesetzt werden.
Die in den Ausführungsbeispielen dargestellten Rohre sind vorzugsweise aus einem im wesentlichen temperaturstabilen Material hergestellt, dessen Dielektrizitätskonstante zudem im wesentlichen temperaturunabhängig ist. Vorzugsweise kommt hier Keramik oder Kunststoff, beispielsweise Polykarbonat, zum Einsatz.

Die anhand der Figuren näher beschriebene Erfindung ist nicht auf diese Ausführungsformen beschränkt. Insbesondere kann der Resonatorraum verschiedene Geometrien anstelle einer zylindrischen Ausgestaltung aufweisen. Weiterhin kann das Führungsrohr auch mit anderen Bandführungselementen anstelle oder zusätzlich zum Bandtrichter ausgebildet sein, beispielsweise als dem Resonatorraum vorgeschaltetes Vliesformungselement. Ebenfalls kann die erfindungsgemäße Vorrichtung auch dem Streckwerk vorgelagert plaziert sein, wobei die maschinentypische Funktionsgruppe zur mechanischen Integration des Resonators ebenfalls Elemente zur Bandüberwachung, Bandführung und/oder Bandeinfädelung aufweist. Bei einer Karden-Strecken-Kombination kann beispielsweise eine erfindungsgemäße Vorrichtung jeweils am Einlauf und am Auslauf der Strecke angeordnet sein.Die Erfindung läßt sich sowohl bei regulierten als auch bei unregulierten Strecken, Karden und Kämmmaschinen einsetzen.

## Patentansprüche

1. Vorrichtung mit einem Mikrowellenresonator für eine oder an einer Spinnereivorbereitungsmaschine, die ein Streckwerk (2) zum Verstrecken von strangförmigem Fasermaterial (FB) aufweist, insbesondere Karde, Strecke oder Kämmmaschine, wobei der Resonator (30) zum Anschießen an eine Messeinrichtung (16) zur Messung der Banddichte bzw. der Banddicke und/oder der Feuchtigkeit des kontinuierlich durch den Resonatorraum (31) hindurchgeförderten Fasermaterials (FB) ausgebildet ist, **dadurch gekennzeichnet, dass** der Resonator (30) in eine maschinentypische Funktionsgruppe (20; 120; 220; 320; 420) der Spinnereivorbereitungsmaschine integriert ist, wobei die Funktionsgruppe (20; 120; 220; 320; 420) eine Trägerkonstruktion (21; 121; 221; 321; 421) umfasst, an der Elemente (23,26,50,51.60;160,126;423,424,460,426;560a, 560b; 660a, 660b) zur Bandüberwachung, Bandführung und/oder Bandeinfädelung angeordnet oder anordenbar sind.

2. Vorrichtung nach Anspruch 1, **dadurch gekennzeichnet, dass** die Funktionsgruppe (20; 120; 220; 320; 420) am Streckwerksausgang angeordnet ist.

3. Vorrichtung nach einem der vorhergehenden Ansprüche, **dadurch gekennzeichnet, dass** eine Vliesführungsdüse (23; 423) an der Trägerkonstruktion (21; 121; 221; 321; 421) angeordnet oder anordenbar ist.

4. Vorrichtung nach einem der vorhergehenden Ansprüche, **dadurch gekennzeichnet**, das ein Bandtrichter (26) und/oder eine Halterung für einen Bandtrichter an der Trägerkonstruktion (21) angeordnet oder anordenbar ist.

5. Vorrichtung nach einem der vorhergehenden Ansprüche, **dadurch gekennzeichnet**, das ein Bandstausensor (50) an der Trägerkonstruktion (21; 121; 221; 321; 4221) angeordnet oder anordenbar ist.

6. Vorrichtung nach einem der vorhergehenden Ansprüche, **dadurch gekennzeichnet**, das der Resonatorraum (31) zylindrisch ausgebildet ist und in Faserbandquerrichtung größere Ausmaße besitzt als in Faserbändfaufrichtung.

7. Vorrichtung nach einem der vorhergehenden Ansprüche, **dadurch gekennzeichnet**, das mindestens ein Anschluss (58, 59) zum Ein- und/oder Auskoppeln der Mikrowellen in den Resonatorraum (31) auf der das Fasermaterial zuführenden Seite des Resonators (30) angeordnet ist.

8. Vorrichtung nach einem der vorhergehenden Ansprüche, **dadurch gekennzeichnet**, das mindestens ein Anschluss (58, 59) zum Ein- und/oder Auskoppeln der Mikrowellen in den Resonatorraum (31) auf der den Resonator (30) verlassenden Seite des Resonators (30) angeordnet ist.

9. Vorrichtung nach einem der vorhergehenden Ansprüche, **dadurch gekennzeichnet**, das mindestens ein Anschluss (58, 59) zum Ein- und/oder Auskoppeln der Mikrowellen in den Resonatorraum (31) seitlich am Resonator (30) angeordnet ist.

10. Vorrichtung nach einem der vorhergehenden Ansprüche, **dadurch gekennzeichnet**, das der Resonator (30) als separates Teil der Funktionsgruppe (20; 120; 220; 320; 420) ausgebildet ist.

11. Vorrichtung nach einem der Ansprüche 1 bis 9, **dadurch gekennzeichnet, dass** die Funktionsgruppe (20; 120; 220; 320; 420) mit zumindest einem Teil des Resonators (30) einstückig ausgebildet ist.

12. Vorrichtung nach einem der vorhergehenden Ansprüche, **dadurch gekennzeichnet, dass** der Resonatorraum (31) zumindest zum Teil von einer Vertiefung (32; 332; 432) in der Funktionsgruppe (20; 320; 420) gebildet ist.

13. Vorrichtung nach einem der vorhergehenden Ansprüche, **gekennzeichnet durch** mindestens ein abnehmbares Wandelement (46; 146, 147; 246, 247; 346, 347; 446) zur Bildung mindestens eines Teils des Resonatorraums (31).

14. Vorrichtung nach Anspruch 12 und 13, **dadurch gekennzeichnet, dass** ein Wandelement (46; 346; 446) zum Aufsetzen auf die Vertiefung (32; 332; 432) ausgebildet ist.

15. Vorrichtung nach einem der vorhergehenden Ansprüche, **dadurch gekennzeichnet, dass** die Funktionsgruppe (120; 220) eine Durchbrechung aufweist auf die von der einen und der anderen Seite ein erstes bzw. ein zweites Wandelement (146, 147; 246, 247) aufgesetzt sind, zwischen denen der Resonatorraum (31) gebildet ist.

16. Vorrichtung nach Anspruch 15, **dadurch gekennzeichnet, dass** die Funktionsgruppe (120) eine umlaufende Seitenwand des Resonatorraums (31) und die beiden abnehmbaren Wandelemente (146, 147) dessen Deckenwand bzw. Bodenwand bilden.

17. Vorrichtung nach einem der Ansprüche 1 bis 15, **dadurch gekennzeichnet, dass** alle Wände des Resonatorraums (31) durch mindestens zwei abnehmbare Wandelemente (246, 247; 346, 347) gebildet sind.

18. Vorrichtung nach einem der vorhergehenden Ansprüche, **gekennzeichnet durch** mindestens einelektrisch nicht leitendes und an beiden Stirnseiten offenes und zur Hindurchführung des Fasermaterials (FB) ausgebildetes Führungsrohr (60; 160; 460; 560a, 560b; 660a, 660b) zum Einsetzen in den Resonatorraum (31).

19. Vorrichtung nach Anspruch 18, **dadurch gekennzeichnet, dass** das mindestens eine Führungsrohr (460; 560a, 560b; 660a, 660b) zumindest abschnittsweise konisch ausgebildet ist.

20. Vorrichtung nach Anspruch 18 oder 19, **dadurch gekennzeichnet, dass** das mindestens eine Führungsrohr (460; 560a, 560b; 660a, 660b) eine erweiterte, vorteilhafterweise konisch ausgebildete, Bandeintrittsöffnung (461; 561b; 661b) aufweist.

21. Vorrichtung nach einem der Anspruche 18 bis 20, **dadurch gekennzeichnet, dass** im Bereich des stromabwärtigen Endes des mindestens einen Führungsrohrs (60; 160; 460; 560a; 660a) ein mit der zugehörigen stirneeitigen Rohröffnung fluchtender Bandtrichter (26; 126; 426; 526; 626) angeordnet oder anordenbar ist.

22. Vorrichtung nach Anspruch 21, **dadurch gekennzeichnet, dass** das mindestens eine Führungsrohr (160; 460; 560a; 660a) und der Bandtrichter (126; 426; 526; 626) einstückig ausgebildet sind.

23. Vorrichtung nach einem der Ansprüche 18 bis 22, **dadurch gekennzeichnet, dass** verschiedene alternativ in den Resonator (30) einsetzbare Führungsrohre (60; 160; 460; 560b; 660b) vorgesehen sind, die jeweils im wesentlichen den gleichen Außendurchmesser und voneinander verschiedene innendurchmesser aufweisen.

24. Vorrichtung nach einem der Ansprüche 18 bis 23, **dadurch gekennzeichnet, dass** mindestens zwei in den Resonator (30) einsetzbare Führungsrohre (560a, 560b; 660a, 660b) vorgesehen sind, wobei ein äußeres Führungsrohr (560; 660a) ein inneres (560b; 660b) umgibt.

25. Vorrichtung nach Anspruch 24, **dadurch gekennzeichnet, dass** verschiedene innere Führungsrohre (560b; 660b) mit unterschiedlichem Innendurchmesser in das äußere Führungsrohr (560a; 660a) einsetzbar sind.

26. Vorrichtung nach einem der Ansprüche 23 bis 25, **dadurch gekennzeichnet, dass** verschiedene, alternativ in den Resonatorraum (31) einsetzbare Führungsrohre (560b; 660b) mit unterschiedlichern Innendurchmesser im wesentlichen die gleiche Masse im Bereich der Mikrowehehausbreitung aufweisen.

27. Vorrichtung nach einem der Ansprüche 18 bis 26, **dadurch gekennzeichnet, dass** das mindestens eine Führungsrohr (60; 160; 460; 560a, 560b; 660a, 660b) aus einem im wesentlichen temperaturstabilen Material hergestellt ist, dessen Dielektrizitätskonstante zudem im wesentlichen temperaturunabhängig ist, vorzugsweise aus Keramik oder Kunststoff, beispielsweise Polykarbonat.

28. Vorrichtung nach einem der vorhergehenden Ansprüche, **dadurch gekennzeichnet, dass** die Funktionsgruppe (420) im Bereich des Resonators (30) auf eine im wesentlichen konstante Temperatur temperierbar ist.

29. Vorrichtung nach Anspruch 28, **gekennzeichnet durch** mindestens eine im Bereich des Resonators angeordnete elektrische Heizfolie (80, 85).

30. Vorrichtung nach Anspruch 28 oder 29, **dadurch gekennzeichnet, dass** ein thermisch isolierendes Material im Bereich der Funktionsgruppe (20; 120; 220; 320) angeordnet ist.

31. Vorrichtung nach einem der vorhergehenden Ansprüche, **dadurch gekennzeichnet, dass** die Funktionsgruppe (20; 120; 220; 320; 420), insbesondere der Resonator (30), zumindest teilweise aus einem Material mit geringem Wärmeausdehnungskoeffizient gefertigt ist.

32. Vorrichtung nach Anspruch 31, **dadurch gekennzeichnet, dass** das Material ein Stahl mit hohem Nickel-Ahteil ist, beispielsweise Invar-Stahl.

33. Vorrichtung nach einem der vorhergehenden Ansprüche, **dadurch gekennzeichnet, dass** der Resonator (30) innenwandig mit einer leitfähigen Beschichtung (90) versehen ist, vorzugsweise einer sauerstoffarmen Kupfer-Beschichtung.

34. Vorrichtung nach einem der vorhergehenden: Ansprüche, **dadurch gekennzeichnet, dass** eine korrosionsbeständige Beschichtung (92) vorgesehen ist, insbesondere eine Gold-oder Silber-Beschichtung, die über einer leitfähigen Beschichtung nach Anspruch 34 aufgebracht sein kann.

35. Vorrichtung nach einem der vorhergehenden Ansprüche, **dadurch gekennzeichnet, dass** der Resonatorraum (31) gasdicht mit einem vor Korrosion schützenden Gas gefüllt ist, insbesondere Edelgas.

36. Spinnereivorbereitungsmaschine, insbesondere Karde, Strecke oder Kämmmaschine, **gekennzeichnet durch** eine Vorrichtung nach einem der vorhergehenden Ansprüche.

## Claims

1. Device with a microwave resonator for or on a spinning mill preparation machine with drafting equipment (2) for drawing skein-shaped fibre material FB, in particular a card, draw frame or combing machine, whereby the resonator (30) is designed for connection to a measuring device (16) to measure the sliver density or sliver thickness respectively and/or the moisture of the fibre sliver (FB) being conveyed continuously through the resonator chamber (31), **characterized in that** the resonator (30) is integrated into a functional group (20; 120; 220; 320; 420) of the spinning preparation machine, said functional group being typical for the machine and comprising a supporting structure (21; 121; 221; 321; 421) on which elements (23, 26, 50, 51, 60; 160, 126; 423, 424, 460, 426; 560a, 560b; 660a, 660b) for sliver monitoring, sliver guidance and/or sliver threading are installed or can be installed.

2. Device according to claim 1, **characterized in that** the functional group (20; 120; 220; 320; 420) is located at the output of the drafting system.

3. Device according to one of the preceding claims, **characterized in that** a fibrous-web guiding nozzle (23; 423) is installed on the supporting structure (21; 121; 221; 321; 421) or can be installed on it.

4. Device according to one of the preceding claims, **characterized in that** a sliver funnel (26) and/or a holder of a sliver funnel is installed or can be installed on the supporting structure (21).

5. Device according to one of the preceding claims, **characterized in that** a sliver pile-up sensor is installed or can be installed on the supporting structure (21; 121; 221; 321; 421).

6. Device according to one of the preceding claims, **characterized in that** the resonator chamber (31) is of cylindrical shape and has larger dimensions in the direction perpendicular to the fibre sliver than in the direction of fibre movement.

7. Device according to one of the preceding claims, **characterized in that** at least one connection (58, 59) for the coupling and/or uncoupling of the microwaves into the resonator chamber (31) is installed on the side of fibre material arrival of the resonator (30).

8. Device according to one of the preceding claims, **characterized in that** at least one connection (58, 59) for coupling and/or uncoupling of the microwaves into the resonator chamber (31) are installed on the output side of the resonator (30).

9. Device according to one of the preceding claims, **characterized in that** at least one connection (58, 59) for the coupling and/or uncoupling of the microwaves into the resonator chamber (31) is installed laterally on the resonator (30).

10. Device according to one of the preceding claims, **characterized in that** the resonator (30) is made as a separate part of the functional group (20; 120; 220; 320; 420).

11. Device according to one of the claims 1 to 10, **characterized in that** the functional group (20; 120; 220; 320; 420) is made in one piece with at least part of the resonator (30).

12. Device according to one of the preceding claims, **characterized in that** the resonator chamber (31) is constituted at least in part by a depression (32; 332; 432) in the functional group (20; 320; 420).

13. Device according to one of the preceding claims, **characterized by** at least one removable wall element (46; 146, 147; 246, 247; 346, 347; 446) for the forming of at least part of the resonator chamber (31).

14. Device according to claim 13 and 14, **characterized in that** a wall element (46; 346; 446) is designed to be set on the depression 32; 332; 432).

15. Device according to one of the preceding claims, **characterized in that** the functional group (120; 220) is provided with an opening on which a first and a second wall element (146, 147; 246, 247) is placed from one side and the other side, with the resonator chamber (31) formed between them.

16. Device according to claim 15, **characterized in that** the functional group (120) constitutes a surrounding lateral wall of the resonator chamber (31) and the two removable wall elements (146, 147) constitute its top and bottom walls.

17. Device according to one of the claims 1 to 15, **characterized in that** all the walls of the resonator chamber (31) are formed by at least two removable wall elements (246, 247; 346, 347)

18. Device according to one of the preceding claims **characterized by** at least one electrically non-conductive guide pipe (60; 160; 460; 560a, 560b; 660a, 660b) open at both ends and designed for the fibre material (FB) to be conveyed through it, for insertion in the resonator chamber (31).

19. Device according to claim 18, **characterized in that** the at least one guide pipe (460; 560a, 560b; 660a, 660b) is at least in part conical.

20. Device according to claim 18 or 19, **characterized in that** the at least one guide pipe (460; 560a, 560b; 660a, 660b) is provided with a widened, advantageously conical sliver input opening (461; 561b; 661b).

21. Device according to one of the claims 18 to 20, **characterized in that** a sliver funnel (26; 126; 426; 526; 626) which is aligned with the appertaining pipe opening at the front end is installed or can be installed in the area of the downstream end of the at least one guide pipe (60; 160; 460; 560a, 660a).

22. Device according to claim 20, **characterized in that** the at least one guide pipe (160; 460; 560a, 660a) and the sliver funnel (126; 426; 526; 626) are made in one piece.

23. Device according to one of the claims 18 to 22, **characterized in that** different guide pipes (60; 160; 460; 560b; 660b) alternatively insertable into the resonator (30) are provided, all having essentially the same outside diameter and different inside diameters each.

24. Device according to one of the claims 18 to 23, **characterized in that** at least two of the guide pipes (560a, 560b; 660a, 660b) insertable into the resonator (30) are provided, whereby an outer guide pipe (560; 660a) surrounds an inner one (560b; 660b).

25. Device according to claim 24, **characterized in that** different inside guide pipes (560b; 660b) with different inside diameters can be inserted into the outside guide pipe (560a; 660a).

26. Device according to one of the claims 23 to 25, **characterized in that** different guide pipes (560b; 660b) with different inside diameters and alternatively insertable into the resonator chamber (31) have essentially the same mass in the area of microwave dispersion.

27. Device according to one of the claims 18 to 26, **characterized in that** the at least one guide pipe (60; 160; 460; 560a, 560b; 660a, 660b) is made of an essentially temperature-stable material the permittivity of which is furthermore essentially independent of temperature, preferably of a ceramic or synthetic material, e.g. polycarbonate.

28. Device according to one of the preceding claims, **characterized in that** the functional group (420) can be brought to an essentially constant temperature in the vicinity of the resonator (30).

29. Device according to claim 28, **characterized by** at least one electric heating foil (80, 85) arranged in the area of the resonator.

30. Device according to claim 28 or 29, **characterized in that** a thermally insulating material is arranged in the area of the functional group (20; 120; 220; 320).

31. Device according to one of the preceding claims, **characterized in that** the functional group (20; 120; 220; 320; 420), in particular the resonator (30), is made at least in part of a material with a low heat expansion coefficient.

32. Device according to claim 31, **characterized in that** the material is a steel with high nickel content, e.g. Invar® steel.

33. Device according to one of the preceding claims, **characterized in that** the resonator (30) is provided on its interior wall with a conductive coating (90), preferably a low-oxygen copper coating.

34. Device according to one of the preceding claims, **characterized in that** a corrosion-proof coating (92) is provided, in particular a gold or silver coating, that can be applied over the conductive coating according to claim 33.

35. Device according to one of the preceding claims, **characterized in that** the resonator chamber (31) is filled in a leak-proof manner with a gas protecting from corrosion in particular inert gas.

36. Spinning mill preparation machine, in particular card, draw frame or combing machine, **characterized by** a device according to one of the preceding claims.

## Revendications

1. Dispositif avec un résonateur à hyperfréquences pour une machine de préparation du filage ou à une machine de préparation du filage, qui comporte un banc d'étirage (2) pour l'étirage de matière fibreuse (FB) en boyau, particulièrement une cardeuse, une étireuse ou une peigneuse, sachant que le résonateur (30) se présente sous une forme permettant de le connecter à un dispositif de mesure (16) pour la mesure de la densité de bande ou de l'épaisseur de bande et/ou de l'humidité de la matière fibreuse (FB) transportée continuellement à travers l'espace de résonateur (31), **caractérisé en ce que** le résonateur (30) est intégré dans un groupe fonctionnel (20; 120; 220; 320; 420) typique à la machine de préparation du filage, sachant que le groupe fonctionnel (20; 120; 220; 320; 420) comporte une construction porteuse (21; 121; 221; 321; 421), à laquelle sont disposés ou peuvent être disposés des éléments (23, 26, 50, 51, 60; 160, 126; 423, 424, 460, 426; 560a, 560b; 660a, 660b) pour la surveillance de la bande, le guidage de la bande et/ou l'enfilage de la bande.

2. Dispositif selon la revendication 1, **caractérisé en ce que** le groupe fonctionnel (20; 120; 220; 320; 420) est disposé à la sortie du banc d'étirage.

3. Dispositif selon l'une quelconque des revendications précédentes, **caractérisé en ce qu'**une buse de guidage de toison (23; 423) est disposée ou peut être disposée à la construction porteuse (21; 121; 221; 321; 421).

4. Dispositif selon l'une quelconque des revendications précédentes, **caractérisé en ce qu'**un entonnoir (26) et/ou une bride de support pour un entonnoir est disposé ou peut être disposé à la construction porteuse (21).

5. Dispositif selon l'une quelconque des revendications précédentes, **caractérisé en ce qu'**un capteur de bourrage de ruban (50) est disposée ou peut être disposé à la construction porteuse (21; 121; 221; 321; 421).

6. Dispositif selon l'une quelconque des revendications précédentes, **caractérisé en ce que** l'espace de résonateur (31) se présente sous une forme cylindrique et qu'il présente des dimensions plus importantes dans le sens transversal au ruban de fibres que dans le sens de translation du ruban de fibres.

7. Dispositif selon l'une quelconque des revendications précédentes, **caractérisé en ce qu'**au moins un raccord (58, 59) pour coupler et/ou découpler les microondes dans l'espace de résonateur (31) est disposé sur le côté du résonateur (30) amenant la matière fibreuse.

8. Dispositif selon l'une quelconque des revendications précédentes, **caractérisé en ce qu'**au moins un raccord (58, 59) pour coupler et/ou découpler les microondes dans l'espace de résonateur (31) est disposé sur le côté du résonateur (30) quittant le résonateur (30).

9. Dispositif selon l'une quelconque des revendications précédentes, **caractérisé en ce qu'**au moins un raccord (58, 59) pour coupler et/ou découpler les microondes dans l'espace de résonateur (31) est disposé latéralement au résonateur (30).

10. Dispositif selon l'une quelconque des revendications précédentes, **caractérisé en ce que** le résonateur (30) se présente sous la forme d'une partie séparée du groupe fonctionnel (20; 120; 220; 320; 420).

11. Dispositif selon l'une quelconque des revendications 1 à 9, **caractérisé en ce que** le groupe fonctionnel (20; 120; 220; 320; 420) se présente sous une forme monocorps avec au moins une partie du résonateur (30).

12. Dispositif selon l'une quelconque des revendications précédentes, **caractérisé en ce que** l'espace de résonateur (31) est formé au moins partiellement par un approfondissement (32; 332; 432) dans le groupe fonctionnel (20; 320; 420).

13. Dispositif selon l'une quelconque des revendications précédentes, **caractérisé par** au moins un élément de paroi amovible (46; 146, 147; 246, 247; 346, 347; 446) pour constituer au moins une partie de l'espace de résonateur (31).

14. Dispositif selon les revendications 12 et 13, **caractérisé en ce qu'**un élément de paroi (46; 346; 446) se présente sous une forme permettant sa pose sur l'approfondissement (32; 332; 432).

15. Dispositif selon l'une quelconque des revendications précédentes, **caractérisé en ce que** le groupe fonctionnel (120; 220) comporte une brèche, sur laquelle sont posés respectivement, sur l'un et sur l'autre côté, un premier et un second élément de paroi (146, 147; 246, 247), entre lesquels est constitué l'espace de résonateur (31).

16. Dispositif selon la revendication 15, **caractérisé en ce que** le groupe fonctionnel (120) constitue une paroi latérale circulaire de l'espace de résonateur (31) et les deux éléments de paroi amovibles (146, 147) sa paroi de bassin ou sa paroi de fond.

17. Dispositif selon l'une quelconque des revendications 1 à 15, **caractérisé en ce que** toutes les parois de l'espace de résonateur (31) sont constituées par au moins deux éléments de paroi amovibles (246, 247; 346, 347).

18. Dispositif selon l'une quelconque des revendications précédentes, **caractérisé par** au moins un tube de guidage (60; 160; 460; 560a, 560b; 660a, 660b) non conducteur électrique et ouvert à extrémités, se présentant sous une forme permettant le passage de la matière fibreuse (FB) et destiné à la mise en place dans l'espace de résonateur (31).

19. Dispositif selon la revendication 18, **caractérisé en ce que** l'au moins un tube de guidage (460; 560a, 560b; 660a, 660b) se présente sous une forme conique au moins en certains de ses segments.

20. Dispositif selon l'une quelconque des revendications 18 ou 19, **caractérisé en ce que** l'au moins un tube de guidage (460; 560a, 560b; 660a, 660b) comporte un orifice d'entrée de ruban (461; 561b; 661b) élargi, de préférence conique.

21. Dispositif selon l'une quelconque des revendications 18 à 20, **caractérisé en ce qu'**un entonnoir (26; 126; 426; 526, 626) en alignement précis avec l'orifice de tube correspondant est disposé ou peut être disposé dans la zone de l'extrémité aval de l'au moins un tube de guidage (60; 160; 460; 560a; 660a).

22. Dispositif selon la revendication 21, **caractérisé en ce que** l'au moins un tube de guidage (160; 460; 560a; 660a) et l'entonnoir (126; 426; 526; 626) se présente sous une forme monocorps.

23. Dispositif selon l'une quelconque des revendications 18 à 22, **caractérisé en ce que** divers tubes de guidage (60; 160; 460; 560b; 660b) différents pouvant être mis en place en alternative dans le résonateur (30) sont prévus, lesquels présentent essentiellement le même diamètre extérieur et des diamètres intérieurs différents l'un des autres.

24. Dispositif selon l'une quelconque des revendications 18 à 23, **caractérisé en ce qu'**au moins deux tubes de guidage (560a, 560b; 660a, 660b) pouvant être mis en place dans le résonateur (30) sont prévus, sachant qu'un tube de guidage extérieur (560a, 660a) entoure un tube de guidage intérieur (560b; 660b).

25. Dispositif selon la revendication 24, **caractérisé en ce** des tubes de guidage intérieurs (560b; 660b) différents présentant des diamètres intérieurs différents peuvent être disposés dans le tube de guidage extérieur (560a; 660a).

26. Dispositif selon l'une quelconque des revendications 23 à 25, **caractérisé en ce que** des tubes de guidage (560b; 660b) différents pouvant être disposés en alternative dans le résonateur (31) et possédant des diamètres intérieurs différents présentent essentiellement la même masse dans la zone de dispersion des microondes.

27. Dispositif selon l'une quelconque des revendications 18 à 26, **caractérisé en ce que** l'au moins un tube de guidage (60; 160; 460; 560a, 560b; 0660a; 660b) est fabriqué en un matériau essentiellement stable en température, dont la constante diélectrique est en outre essentiellement indépendante de la température, de préférence en céramique ou en matière plastique, par exemple en polycarbonate.

28. Dispositif selon l'une quelconque des revendications précédentes, **caractérisé en ce que** le groupe fonctionnel (420) peut être tempéré à une température essentiellement constante dans la zone du résonateur (30).

29. Dispositif selon la revendication 28, **caractérisé par** au moins une feuille chauffante électrique (80, 85) disposée dans la zone du résonateur.

30. Dispositif selon l'une quelconque des revendications 28 ou 29, **caractérisée qu'**une matière isolante thermiquement est disposée dans la zone du groupe fonctionnel (20; 120; 220; 320).

31. Dispositif selon l'une quelconque des revendications précédentes, **caractérisé en ce que** le groupe fonctionnel (20; 120; 220; 320; 420), particulièrement le résonateur (30), est fabriqué au moins partiellement en un matériau présentant un coefficient de dilatation thermique faible.

32. Dispositif selon la revendication 31, **caractérisé en ce que** le matériau est un acier présentant une teneur en nickel élevée, par exemple de l'acier invar.

33. Dispositif selon l'une quelconque des revendications précédentes, **caractérisé en ce que** le résonateur (30) est doté sur sa paroi intérieure d'un revêtement conductible (90), de préférence d'un revêtement en cuivre à faible teneur en oxygène.

34. Dispositif selon l'une quelconque des revendications précédentes, **caractérisé en ce qu'**un revêtement (92) résistant à la corrosion est prévu, particulièrement un revêtement en or ou en argent, qui peut être appliqué sur un revêtement conductible selon la revendication 33.

35. Dispositif selon l'une quelconque des revendications précédentes, **caractérisé en ce que** l'espace de résonateur (31) est rempli de manière étanche aux gaz d'un gaz protégeant de la corrosion, particulièrement un gaz noble.

36. Machine de préparation de filage, particulièrement cardeuse, étireuse ou peigneuse, **caractérisée par** un dispositif selon l'une quelconque des revendications précédentes.
